(19) Europäisches Patentamt
European Patent Office
Office européen des brevets

(11) **EP 2 090 330 B1**

(12) **EUROPEAN PATENT SPECIFICATION**

(45) Date of publication and mention
of the grant of the patent:
**27.11.2019 Bulletin 2019/48**

(51) Int Cl.:
*A61M 27/00* (2006.01)    *A61B 5/03* (2006.01)

(21) Application number: **09250354.9**

(22) Date of filing: **12.02.2009**

(54) **Combined pressure and flow sensor integrated in a shunt system**

Kombinierter Druck- und Durchflusssensor in einem Shunt-System

Capteur combiné de pression et d'écoulement intégré dans un système de dérivation

(84) Designated Contracting States:
**CH DE FR GB IT LI**

(30) Priority: **13.02.2008 US 30604**

(43) Date of publication of application:
**19.08.2009 Bulletin 2009/34**

(73) Proprietor: **INTEGRA LIFESCIENCES SWITZERLAND SÀRL**
**2400 Le Locle (CH)**

(72) Inventors:
• **Ginggen, Alec**
**200 Neuchatel (CH)**

• **Tandy, Yanik**
**Geneveys-sur-Coffrane (CH)**

(74) Representative: **Tunstall, Christopher Stephen et al**
**Carpmaels & Ransford LLP**
**One Southampton Row**
**London WC1B 5HA (GB)**

(56) References cited:
**EP-A- 1 050 264    EP-A- 1 491 137**
**WO-A-01/21066**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 090 330 B1

**Description**

BACKGROUND OF THE INVENTION

**Field of the Invention**

The present invention generally relates to devices used in the treatment of hydrocephalus, and more particularly, to improvements to sensors and shunts used to help divert and monitor excess fluid during the treatment therapy.

**Description of the Related Art**

Background:

The human brain includes four ventricles. Each ventricle contains a choroid plexus that produces cerebrospinal fluid (CSF) which bathes and cushions the brain and spinal cord within their bony and nonelastic confines.

In a normal healthy person, CSF continuously circulates through and around the brain and its ventricles and around the spinal cord and is continuously drained away into the circulatory system so that a controlled pressure is continually maintained within the system. The CSF flows from the lateral ventricles via the foramina of Monro into the third ventricle, and then the fourth ventricle via the cerebral aqueduct in the brainstem. From there it normally can pass into the central canal of the spinal cord or into the cisterns of the subarachnoid space via three small foramina: the central foramen of Magendie and the two lateral foramina of Luschka.

The aqueduct between the third and fourth ventricles is very small, as are the foramina and both are therefore susceptable to becoming blocked or restricted, commonly due to a birth defect, or a local growth, such as that caused by a tumor or infection, thereby disrupting the normal CSF flow. When the CSF flow is impeded, the continued production of CSF will cause an increase in intracranial pressure as the fluid collects within the venticles.

Alternatively, a similar increase in intracranial pressure of the patient may result from an overproduction of the CSF fluid, from a congenital malformation, or from complications of head injuries or infections, or in some cases, by malabsorption. In any case, the result is the same, an increase of CSF fluid within the ventricles and an increase in intracranial pressure. This condition is called hydrocephalus.

When the CSF accumulates in the cerebral ventricles, the increased volume of fluid compresses the patient's brain tissue since the patient's skull will not yield to this unplanned expansion of fluid. Unfortunately, this compression destroys more and more brain tissue and a variety of secondary symptoms will become apparent in the patient as the neurological functions effectively shut down. These include, headaches, vomiting, dizziness, slurred speech, photophobia/light sensitivity, and in more severe cases, seizures, loss of consciousness and even death.

Hydrocephalus is often treated by the insertion of a diverting catheter into the ventricles of the brain or into the lumbar cistern. Such a catheter or shunt is connected by a regulating valve to a distal catheter which shunts the CSF to another space where it can be reabsorbed and the excess pressure within the brain released. Examples of common diversion sites include the peritoneum of the abdomen via a ventriculoperitoneal shunt or lumboperitoneal shunt or the atrium of the heart via a ventriculoatrial shunt.

A commonly used shunt to treat hydrocephalus is called the Spitz-Holter shunt. It is a conduit that is positioned between the patient's brain and the patient's heart. The device includes a tiny one-way valve that allows a controlled amount of CSF to leave the lateral ventricle of the the brain and enter the heart and thereby prevent the increased pressure that causes such damage to the tissues of the brain. This device has helped millions survive this potentially fatal condition since the late 1950s.

Although the symptoms of excessive intracranial pressure and associated ventricular enlargement may be relieved by this procedure, it is not uncommon for the shunt apparatus to become obstructed, resulting in shunt failure.

About 50% of the shunts fail within the first 5 years after implantation independent of the shunt operating principle and the hydrocephalus etiology. Such shunt failure requires a revision of the shunt system within the patient to avoid a return of original hydrocephalus symptoms. The main causes of failure are infection of the shunt system, obstruction of the shunt, and over-drainage and under-drainage of CSF within the shunt system.

Over-drainage results in an excessive average flow of CFS through the shunt system. This condition may generate an abnormally low intra-cranial pressure, a collapse of the parenchyma and sub-arachnoid hemorrhage.

Similarly, under-drainage results of an insufficient average flow of CSF through the shunt system. Under-drainage may generate a return of the underlying hydrocephalic condition since, by definition, all of the required fluid is not being removed from the lateral ventricles of the patient's brain. Over and under drainage conditions are signs that the shunt has failed to restore a physiological CSF flowpath and the patient's health continues to be at risk. These factors show, if anything, that a tool allowing for better understanding of the critical parameters of CSF hydrodynamics in patients implanted with a shunt would be very beneficial to successful shunt design, selection and implantation strategy.

Some shunt systems use a so-called "Codman Hakim valve" which is commercially available from Codman & Shurtleff, Inc. of Raynham, MA. The Codman Hakim valve allows a doctor to adjust the valve opening pressure non-invasively after implantation. At least two such valve adjustments are required in more than 25% of the cases. The lack of proper tools to predict an optimal shunt setting forces the physician to follow an empirical adjustment strategy. Based on surgeon experience, the shunt setting is fixed to a value after implantation. If the long-term follow-up of the patient shows symptoms that could be due to over or under drainage, the shunt setting is modified accordingly, until an asymptomatic state is reached. Instead of following a "let's wait and see how things work out" approach, a tool that provides quantitative feedback of the condition of the fluid within the shunt to the physician would greatly improve the valve adjustment strategy. It would also likely improve long-term patient outcome.

As implied above, the two important parameters regarding hydrocephalus are the intracranial pressure (ICP) and the flow rate of CSF through the shunt. The value of the intracranial pressure is a direct indicator of the success (or failure) of a hydrocephalus treatment.

The aim of a shunt system is to restore a physiological flow path for the CSF and a physiological intracranial pressure level. The ability to monitor the intracranial pressure non-invasively after the implantation of a shunt system would provide a direct feedback about the patient state and the success of the therapy.

As mentioned above, the flow rate of the CSF in the shunt system is also a key parameter whose value can confirm that the shunt is operating correctly and is not-blocked. As of today, the physician must rely on the diagnosis of the patient and on costly imaging techniques (e.g., MRI) to assess the state of the patient and/or the size of the ventricles. Although this current method of assessment may provide an insight on the current state of the hydrocephalic condition, and also indirectly, a proper functioning shunt system, this assessment may be misleading. For example, a patient implanted with a shunt who still shows signs of hydrocephalus may be suffering from an obstruction of his shunt system or may be suffering from an inappropriately selected shunt system being implanted (e.g., too high resistance or too high opening pressure) even though the shunt system appears to be operating properly. Therefore, the ability to monitor the CSF flow rate non-invasively after the implantation of a shunt system would provide a direct feedback about the patency of the shunt system.

The combination of the variation of pressure observed when CSF is flowing in the shunt and of the CSF flow rate through the shunt system provides an access to the compliance of the content of the cranial vault (brain tissue and spinal sac in a first approximation). The compliance is a measurement of the elasticity of the brain. The higher the compliance the lower the elasticity.

The compliance C is calculated based on the following equation:

$$C = \partial V / \partial P$$

Where $\partial V$ is the variation of the volume associated with a pressure variation $\partial P$.

The variation of the volume $\Delta V$ is linked to the flow rate in the shunt Q(t) by the following equation, assuming that the variation of volume due to the variation of the blood volume in the cranial vault is negligible:

$$\partial V = \int [ Q ( t ) - Q_{average} ] \partial t$$

Where $Q_{average}$ is the average flow rate in the shunt when the patient is not moving. $Q_{average}$ typically corresponds to the production rate of the CSF assuming that all the produced CSF is evacuated by the shunt system.

Therefore, the compliance equation can be re-written as:

$$C = ( \int [ Q ( t ) - Q_{average} ] \partial t ) / \partial P$$

From this equation, it is clear that brain compliance can be measured based on the flow rate of the CSF in the shunt and the ICP.

The compliance is a parameter that is rarely measured on human patients since it requires a direct access to the brain. With current technologies, the compliance can only be measured by the insertion of a probe directly into the brain (an invasive process). A product allowing such invasive measurement of the ICP and of the compliance is commercially available by Spiegelberg (GmbH & Co.) KG, a company headquartered in Hamberg, Germany. This device inflates and deflates a balloon-tipped catheter that has been inserted into a target site within a patient's brain in a controlled manner to calculate ICP and brain compliance. Unfortunately, the catheter must be invasively inserted into the patient and therefore, brain compliance can only be measured on few and carefully planned occasions.

**Prior Art Sensors**

To help measure the pressure of the CSF, a limited number of pressure sensors have been developed that can be integrated directly into the shunt systems, but the commercial distribution of such pressure sensors has since been discontinued. These sensors include the Cosman sensor, which was sold by Radionics, Inc and a Japanese sensor called the Miyake sensor, which was sold in Japan.

Clinical use of existing sensors has been limited by the following factors: size of the sensor housing, complexity of the interrogation procedure, complexity of the implantation procedure, stability of the sensor reading with time, and lifetime of the sensor. These shortcomings have limited their extended use in a clinical setting.

US Patent Nos 4,676,255; 4,660,568; 4,653,508; 4,593,703; 4,378,809; 4,281,667; 4,206,761 and 4,206,762 of Cosman disclose a sensor that is designed to be implanted within a patient's skull for the purpose of indicating intracranial pressure. This device was one of the first stand-alone, implantable pressure sensors that could be interrogated by telemetry.

The Cosman sensor includes a pressure transducer, a transmitter and controlling circuitry located within a protected silicone rubber capsule. The capsule is attached to the shunt system so that the transducer is exposed to the ICP and to the subcutaneous pressure. The specific details of the device are beyond the scope of this immediate invention, but briefly, in operation, the transducer is capable of measuring the differential pressure between the subcutaneous pressure and the ICP using an L-C circuit (inductive and capacitive). The inductance of the circuit will vary with the differential pressure between the subcutaneous pressure and the ICP. The sensor is interrogated by an external reader which effectively "reads" the frequency of the L-C circuit. Then, a pressurizing cuff is applied on the skin adjacent to the sensor, and its pressure is increased until the subcutaneous pressure equals the intracranial pressure, at which point, the transducer is balanced. The external cuff pressure gives a reading of the average ICP, whereas the variation of the inductance and, therefore, of the resonance frequency of the transducer gives a reading of the short-term variation of the ICP. This system has been commercially distributed and used in multiple clinical applications. Its main limitation is the complexity of the reading process, and also the fact that it relies on a pressurization of the sensor through an external cuff. This factor also limits the use of the sensor for short term measurements.

Another sensor, called the Miyake sensor is based on the same principle as the above-described Cosman sensor, except that the Miyake sensor detects the absolute value of the ICP. However, the transducer concept is also similar in that the relative ICP value to the atmospheric pressure is calculated by an external reading unit based on a built-in barometer, thereby obviating the use of a pressurization cuff as in the Cosman sensor. This sensor has also been distributed commercially and used in multiple clinical applications. The principal limitation of this sensor is its size; it has to be implanted in the burr hole of a patient's skull to minimize protrusion of the device.

Another system, called the Insite system works on a completely different concept. This system is conceptually very similar to a conventional cardiac pacemaker. Here, a transducer is located at the distal end of a catheter which is connected to a titanium capsule, which in turn, contains a battery, and the signal conditioning and telemetry electronics.

The Insite sensor is a stand-alone system and is not attached to a shunt. One key advantage of such a concept is that it allows on-board storage of pressure data that can be downloaded by an external reading unit upon request. The complexity of the implantation procedure and its size are the main limitations of such a system. Also, the shunt and the sensor have to be implanted at two different sites within the patient, and, since the Insite implant requires onboard power (a battery), its useful operational life is limited by the battery's capacity. A prior art apparatus that uses a sensor for monitoring intracranial pressure is known from WO01/21066A. Therein disclosed is an apparatus for monitoring intracranial cerebral spinal fluid pressure in a subject, said apparatus comprising a sensor for sensing intracranial pressure, control means for collecting data from said sensor corresponding to sensed intracranial pressure, a spread-spectrum transmitter, means for communicating said data from said control means to said transmitter, a power source for powering said sensor and said control means, wherein data packets from the sensor data are stored and periodically transferred to said transmitter for transmission to an external telemetry receiver. A prior art system and method that uses acoustically activated transducers to receive and transmit signals into and out of the patient's body is known from EPA1050264. A prior art implantable device that uses a CPU to calculate pressure within a valve is known from EPA1491137.

Although there are several prior art devices and inventions which allow a clinician or physician to measure the ICP within a patient's brain, each of these devices are deficient as described above and none of them are capable of measuring in real-time ICP, rate of flow of CSF through a shunt, and brain compliance.

An object of the present invention is to provide an implantable shunt and a corresponding external reader that overcomes the deficiencies of the prior art.

It is another object of the present invention to provide a system that allows for the real-time measurement of ICP, the rate of flow of CSF of a patient and brain compliance.

It is yet another object of the invention to provide an implantable shunt that includes both a pressure sensor and a rate of flow sensor for the treatment of hydrocephalus.

## Summary of the Invention

Accordingly, the present invention provides a shunt system for implantation into a patient for diverting excess fluid from the lateral ventricles of the patient's brain to a diversion site. The present shunt includes a pressure transducer for measuring ICP, a rate of flow transducer for measuring the flow rate of the CSF flowing within the shunt, and telemetry circuitry. A remote reader with an antenna is provided to simultaneously interrogate and provide inductive power to the implanted shunt. Upon interrogation, the pressure and rate of flow measurements are compensated by the on board calibration parameters, multiplexed and transmitted on a carrier signal back to the remote reader/antenna. The reader/antenna extracts the pressure and flow rate data from the carrier wave and further separates the data by demultiplexing the signal. The reader further includes a barometer for providing local barometric pressure and uses this information together with the data from the implanted transducers to calculate and display real-time adjusted ICP, CSF flow-rate within the shunt and brain compliance.

The accompanying drawings show examples of embodiments of the present invention. They illustrate how the invention achieves the above stated advantages and objectives.

## Brief Description of the Drawings

Fig. 1 is an illustrative view of a shunt implanted within a patient showing a reader and a display for selectively interrogating sensors located within the shunt, according to the present invention;

Fig. 2 is a perspective view of a portion of the shunt, according to the invention, showing details of a valve portion, a sensor portion, and a fluid lumen;

Fig. 3 is a partial enlarged section view of the sensor portion of Fig. 2, showing internal details of a sensor pod including a pressure transducer, a flow transducer, a telemetry coil and controlling circuitry, according to the present invention;

Fig. 4 is a schematic view of the operation and connection of the controlling circuitry, sensors, and telemetry coil of Fig. 3, according to the present invention; and

Fig. 5 is a schematic view of the operation and system connection of the reader of Fig. 1, according to the present invention.

## Detailed Description of the Preferred Embodiments

By way of overview and introduction, the present invention concerns improvements in the shunt that is used as part of a hydrocephalus treatment therapy to divert excess fluid from the lateral ventricles of a patient's brain to a diversion site located elsewhere in the patient's body. As mentioned above in the background section of this patent application, it would be desirable to be able to measure on demand both the pressure and the rate of flow of the CSF within such a fluid-diverting system. It has been shown above that a parameter called "brain compliance" might be a useful value in determining the overall success of the therapy used to treat the patient's hydrocephalus condition and that this parameter can be calculated if the CSF pressure, instantaneous flow rate and steady state flow rate are both known. To this end, the improved shunt of this invention allows for a non-invasive real-time measurement of all three of these parameters. When used together with a reader, further according to this invention, the CSF pressure and flow rate values can then be relayed to an external control unit. This control unit can then be used to calculate and display a real-time value of brain compliance after taking local barometer measurements into account, and further display the CSF pressure and flow rate values. The physician can use this information to adjust the shunt system, if necessary until the system yields desirable readings. Such immediate feedback of these parameters from the shunt, according to the present invention, will ensure a quicker and more successful treatment therapy and will more likely result in less discomfort and less risk to the patient. Reference(s) to "embodiment(s)" throughout the description which are not under the scope of the appended claims merely represent possible exemplary executions and are therefore not part of the present invention.

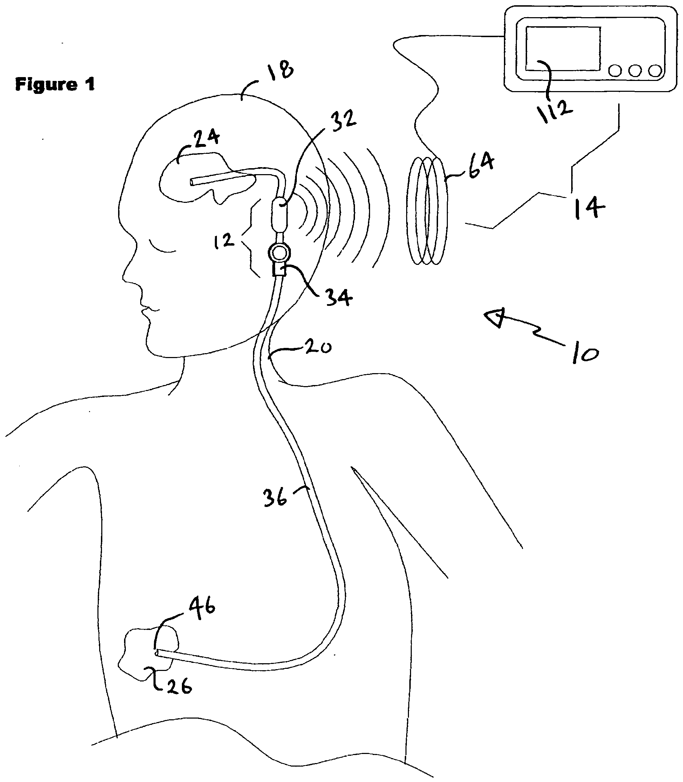

Referring now to Fig. 1, according to the present invention, the shunt assembly 10 includes two main components, a shunt 12 that is designed to be surgically implanted within a patient, and an external reading unit 14. As shown in Fig. 1, an illustrative view of a patient 16 is shown having a head 18, a neck 20, and a torso 22.

For explanatory purposes only, shown in the patient's head 18, is representation of the lateral ventricles 24 of the patient's brain. According to the present invention, shunt 12 is surgically positioned within the patient so that any access CSF can drain from the lateral ventricles 24 to a diversion site 26 located within the patient's body. Common diversion sites include the peritoneum of the abdomen via a ventriculoperitoneal shunt or lumboperitoneal shunt or the atrium of the heart via a ventriculoatrial shunt. The actual surgical procedure for implanting the shunt 12 to provide fluid communication between the lateral ventricle of the patient's brain and the selected diversion site is well known by those skilled in the art and the particular details of the surgical procedure are beyond the scope of the immediate invention.

The present shunt 12 is about the same size and shape as conventional shunts used for this purpose and can therefore be implanted within the patient in the same or a similar manner to the procedure for implanting conventional shunts to treat hydrocephalus. The present invention relates to improvements to the shunt itself and also to a system for communicating with sensors located within the shunt, as described in greater detail below.

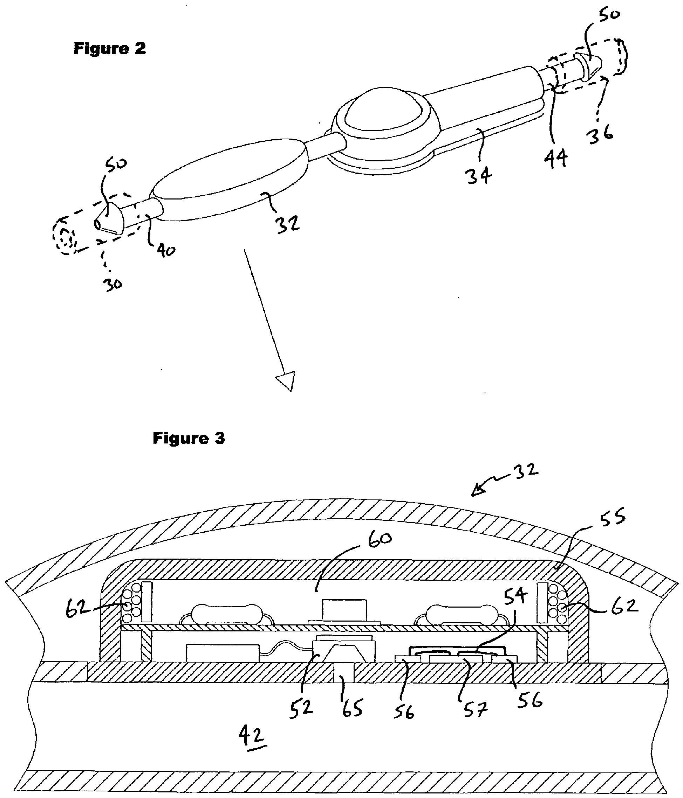

As described above, there is a need to be able to measure both the pressure of the CSF within the shunt 12 and the rate of flow of the CSF within the shunt 12. Knowing these two parameters will allow for the calculation of the brain compliance parameter. Referring now to Figs. 2 and 3, details of shunt 12, according to the present invention are shown. According to the present invention, shunt 12 includes a ventricular catheter 30, a sensor pod 32, a regulating valve 34 and a distal catheter 36.

Referring to Figures 1, 2 and 3, ventricular catheter 30 includes an inlet 36 that is positioned at a desired site within lateral ventricle 24 of the patient (or another desired location), and an outlet 38 that is connected to an inlet 40 of sensor pod 32. As described in greater detail below, sensor pod 32 includes a lumen 42 that is connected to valve 34. Distal catheter 36 is connected to an outlet 44 of valve 34 and includes a discharge port 46 located at a diversion site 26 within the patient. As shown in Fig. 2, sensor pod 32 and valve 34 are shown secured integrally within one package and are shown with nipple fluid connections 50 that are designed to secure to ventricular catheter 30 and distal catheter 36. Although the two catheters are shown as separate components in the shunt system, it is understood that both ventricular catheter 30 and distal catheter 36, valve 34 and sensor pod 32 may all be permanently secured to each other prior to being implanted within the patient using any appropriate method, such as ultrasonic welding, or using an appropriate adhesive.

Regardless of how the shunt assembly is constructed, it is important that sensor pod 32 be positioned proximal to regulating valve 34 (i.e., between ventricular catheter 30 and the valve) so that the sensors contained within are directly exposed to the pressure within the brain through ventricular catheter 30. Also, since it is likely that sensor pod 32 and valve 34 will be positioned against the patient's skull, under the scalp, it is desirable to package the components to provide as low a profile as possible. Much of what is shown in the figures is to help explain the operation and structure of the present invention. It is understood that the components shown and described can fit into any of a variety of appropriate packages as understood by those skilled in the art.

When operating within a patient, the shunt system will allow excess CSF located within the lateral ventricle 24 of the patient's brain to enter the ventricular catheter 30, pass through lumen 42 of sensor pod 32, pass through valve 34, and through distal catheter 36 to eventually be discharged at the diversion site 26.

The purpose of regulating valve 34 is to physically control the flow of fluid passing through the shunt, and therefore the ICP. Typically this valve is a one-way type, allowing fluid to only pass from the lateral ventricle 24 to the diversion site 26, and is also often adjustable from a remote site outside the patient by a physician as necessary to control the flow. The particulars of regulating valve 34 is beyond the scope of the present invention and therefore its operation and structure are not provided here in any great detail.

Referring now to Fig. 3, and in accordance with the present invention, sensor pod 32 includes two transducers: a pressure transducer 52 and a flow-rate transducer 54, both of which are located within a protective housing 55. As is described in greater detail below, these two transducers convert a physical parameter of the fluid flowing within the shunt assembly into an electrical parameter. For example, pressure transducer 52 converts the pressure value into a capacitance value, and flow-rate transducer 54 converts the flow rate into a resistance value. Transmitting electronics selectively transmit sensor data to a location outside the patient's body, when interrogated by an external reader.

To provide accurate readings, pressure transducer 52 is preferably positioned within sensor pod 32 so that its sensor input is in direct fluid communication with the CSF located within adjacent lumen 42. Pressure transducer 52 is preferably a capacitive-type pressure transducer since this type of transducer is capable of measuring pressure with a high degree of accuracy with very low drift characteristics and requires very low power to operate. Additionally, capacitive-type pressure transducers are capable of measuring absolute pressure, independent of atmospheric pressure.

Flow-rate transducer 54 is preferably the type that uses a thermal anemometer, such as the one shown in U.S. Patents Nos. 7,069,779; 7181963; and 7036369, which are hereby incorporated by reference or using a similar thermal anemometer commercially available by Swiss company Sensirion, Inc., which has offices in the US at Westlake Village, CA. These thermal anemometers operate similarly, but the above version that is disclosed in U.S. Patents Nos. 7,069,779; 7181963; and 7036369 is used to correlate the flow rate of a passing fluid by measuring temperature gradients across thermal micro-sensor elements 56 (as the passing fluid cools, or heats the local region) that are located along the direction of the fluid flow. The fluid contacts the sensor surface, either directly or through a thin protective wall, such as the wall of housing 55. The sensor includes a heating element 57, which is disposed between or adjacent to one or more micro-sensor elements 56. The micro-sensor elements are temperature sensitive. In operation, the heat generated by heating element 57 is transferred to one or more of the temperature sensitive micro-sensor elements 56 and the amount of heat energy received by each micro-sensor element 56 is measured, thereby defining a baseline stable system (prior to fluid flow). As fluid flows next to or near the sensor components, the passing fluid will draw heat away from the local sensor system and thereby affect the amount of heat energy from heating element 57 that is received by the micro-sensor elements 56. The amount of heat transferred is directly correlated to the fluid flow, as the heat is partially dissipated in the fluid.

Both pressure transducer 52 and flow-rate transducer 54 are connected to an electronic processing circuit 60 that provides signal conditioning of the outputs signals provided during the pressure and flow-rate measurements. Electronic processing circuit 60 is also connected to a loop antenna 62 that allows the simultaneous communication of power and of data between a nearby interrogating antenna 64 (see Figure 1), as described below. Both transducers, 52, 54, controlling processing circuitry 60 and loop antenna 62 are all contained with housing 55. A bore 65 is formed within a wall of housing 55 so that CSF may contact pressure transducer 52, as necessary to allow the pressure of the CSF within the shunt to be directly measured. The CSF is allowed to enter bore 65 and interact directly with pressure transducer 52, as-necessary, but is otherwise sealed from the other components within housing 55 using appropriate well known sealing techniques.

Although pressure transducer 52 and flow-rate transducer 54 are described and shown as being separate components located on a circuit board located within housing 55, it should be understood that it would be quite feasible to provide both the pressure transducer 52 and the flow-rate transducer 54 onto a single semiconductor substrate, including other subsystems of this invention, such as the telemetry circuitry, memory, and microprocessor circuitry. Loop antenna 62 is preferably formed from a coil of conductive material such as metallic wire, carbon fiber wires, conductive ink, conductive elastomeric materials, or other conventional inductor materials.

**The Transponder:**

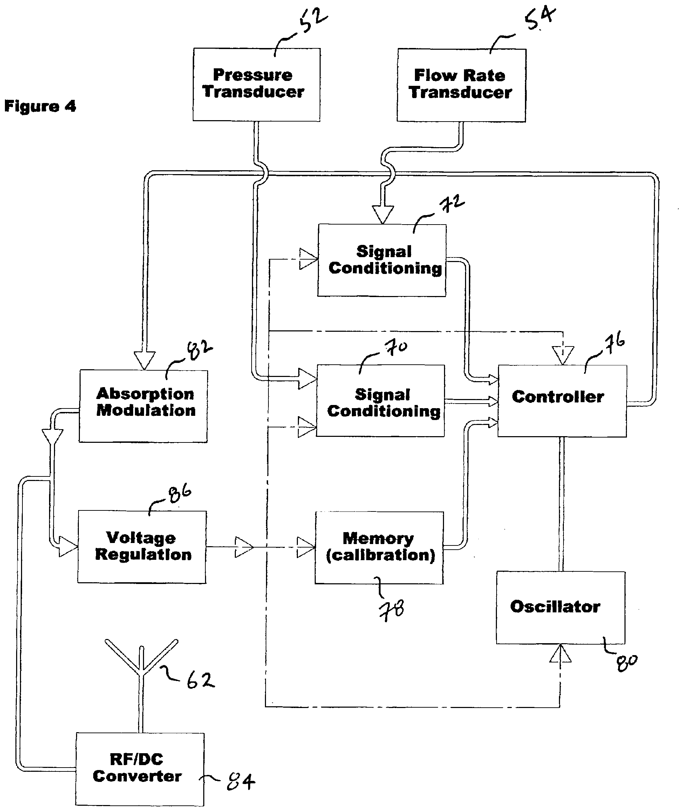

Referring now to Fig. 4, a block diagram is shown to illustrate the functional operation and electrical interaction of the processing circuitry 60, the connected transducers 52, 54, and the loop antenna 62, according to the invention.

The signal output of pressure transducer 52 is inputted to a signal conditioning circuit 70. Similarly, the signal output of flow-rate transducer 54 is inputted to a signal conditioning circuit 72. The purpose of the signal conditioning circuits 70, 72 is to convert the analog signal of both the pressure transducer 52 and the flow-rate transducer 54 to respective digital signals. This type of analog/digital conditioning is well known to those skilled in the art.

The now digital output of each signal conditioning circuits 70, 72 is inputted to a controller circuit 76, which is essentially a microprocessor, but may be a microcontroller or a digital signal processor. Also connected to controller circuit 76 is a memory 78 storing calibration information that is unique to the particular transducers used in the particular shunt assembly. The calibration information from memory 78 is used by controller circuit 76 to adjust and correct the output data of each transducer, 52, 54. As is well known in the art, an oscillator circuit 80 is connected to controller circuit 76 to provide a clocking signal that is necessary for a microprocessor, such as controller circuit 76 to function.

As shown in Fig. 4, controller block 76 is connected to an absorption modulation block 82. Prior to outputting the now calibration adjusted and digitized data of both transducers, controller block 76 first multiplexes the data. Controller block 76 further controls the absorption modulation block 82, whose function is to encrypt the multiplexed transducer data onto a carrier frequency signal that is then sent to loop antenna 62. Since multiplexing, digitizing and encrypting data onto carrier signals to be transmitted on a radio frequency are all well known techniques by those skilled in the art, the circuitry details required to accomplish these tasks are not described here in any great detail.

Loop antenna 62 is connected to RF/DC converter block 84 that converts the incoming RF wave to a DC signal. This signal is supplied to the voltage regulation block 86 which regulates and supplies power for all of the components that make up processing circuitry 60 (the dashed lines of Fig. 4 represent the voltage supply lines). There are several known modulation schemes to both transmit data and simultaneously transmit inductive power to an implanted device from a single mated coil pair. Among these schemes are load-shift keying, phase-shift keying, and frequency-shift keying and amplitude-shift keying. These and other modulation techniques are described in an article entitled: "Data Transmission from An Implantable Biotelemeter by Load-Shift Keying Using Circuit Configuration Modulator," by Zhengnian Tang, Brian Smith, John H. Schild, and P. Hunter Peckham, published in the IEEE Transaction on Biomedical Engineering, Volume 42, No. 5, May, 1995. This article is hereby incorporated by reference. Such shift keying modulation allows simultaneous powering or energizing of an implanted transponder and data transmission from the transponder through the same radio frequency (RF) inductive couple. Use of an induction coil to power the on-board components obviates the need for on-board batteries and thereby effectively extends the useful life of the present device and makes for a more compact assembly.

Although it is preferred to have the implanted components be powered by a remotely applied RF energy (i.e.,

passively), Applicants still contemplate providing a local power supply, such as an onboard battery. In such instance, the telemetry system could actively transmit a locally generated RF wave to communicate with a remote reader. Other methods could be employed to relay the data of the implant to a remote device without departing from the invention.

**The Reader:**

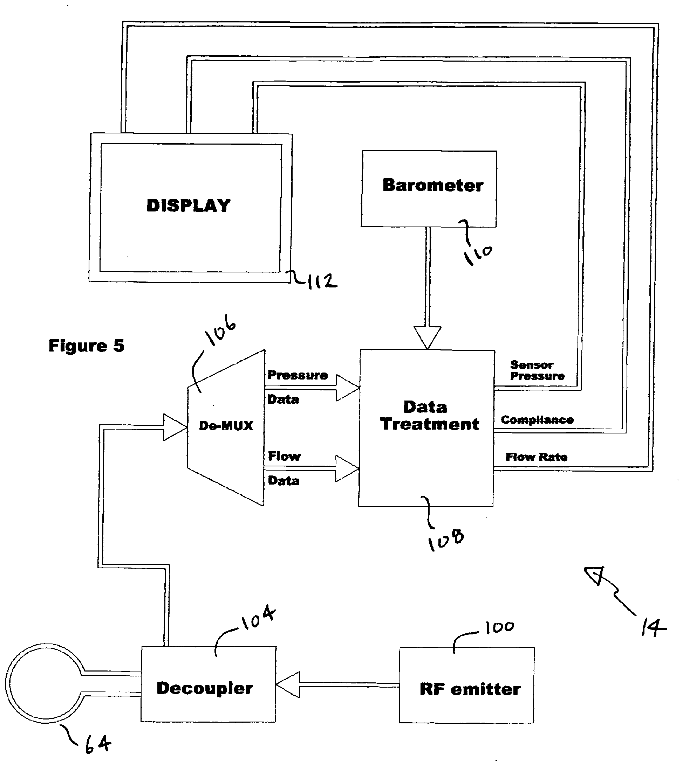

Referring now to Fig. 5, a block diagram is shown to illustrate the functional operation and electrical interaction of the components that make up external reading unit 14, according to the invention.

External reading unit 14 includes an RF emitter 100 which is connected to interrogation antenna 64 through a decoupler block 104. RF emitter is used to generate an RF carrier wave that is transmitted through interrogation antenna 64. When interrogation antenna 64 is in the vicinity of loop antenna 62, the transmitted RF carrier wave is sufficient in effective strength to bring the processing circuit 60 of the shunt 12 to life. When data is transmitted back to interrogation antenna 64 on the return carrier wave, decoupler block 104 is used to separate or de-couple the encrypted data signal from the carrier wave.

Decoupler block 104 is connected to a demultiplexer block 106 which receives as an input the separated multiplexed data signals from decoupler block 104. Demultiplexer block 106 is used to separate (or demultiplex) the received multiplexed data signals into the original digital and calibrated pressure and flow data signals. These separated signals are then inputted to a data treatment block 108, along with data from a barometer block 110. Barometer block 110 measures and sends current barometric pressure to data treatment block 108 so that accurate ICP and compliance calculations can be made.

Data treatment block 108 uses the inputted information from barometric block 110 and from Demultiplexer block 106 to calculate:

**1) Adjusted Intracranial Pressure (ICP) Values**

The adjusted ICP is the result of the subtraction of atmospheric pressure measured by barometer block 110 from the absolute pressure measured by the implanted pressure transducer 52.

**2) The Flow-Rate of CSF** (within the shunt)

This is a pretty straight-forward calculation since data treatment merely has to display the cleaned-up data signal it receives from the shunt. The displayed value of CSF will be a real time measurement by the flow-rate transducer 54. However, this value will likely fluctuate considerably as the patient moves, lies down or sits upright.

**3) The Compliance of the Intra-Cranial Vault;**

This parameter is calculated based on Equation:

$$C = \left( \int [ Q ( t ) - Q_{average} ] \partial t \right) / \partial P$$

also described above.

The average flow rate in the shunt (steady-state situation) has first to be measured, when the patient is not moving, and when the blood volume in the brain is not significantly changing (i.e the arterio-venous flow has to be stable). This situation corresponds to an equilibrium situation where the CSF production rate is compensated by the CSF absorption rate and the CSF flow rate through the shunt system. The volume of the brain, of the arterio-venous system and of the ventricular system is constant in average. Then, a maneuver has to be performed in order to modify the equilibrium situation.

One could envision that the physician activates the pumping chamber of the valve. This will increase the flow rate in the shunt system and decrease the pressure. The above equation allows the calculation of the compliance of the cranio-spinal vault. Another simple maneuver is a change in the patient position (e.g. from supine to standing). This will also increase the flow rate in the shunt and decrease the intra-cranial pressure.

Data treatment block 108 further calculates flow-rate of the CSF (both instantaneous and average) within the shunt, and brain-compliance using the above-shown formula. The information is then communicated to display 112 using conventional display-driver and display technologies. The displayed values of ICP, flow-rate of CSF and brain compliance are real-time values and are invaluable to the physician or clinician performing shunt valve adjustment or diagnosis and evaluation of the hydrocephalus treatment.

The sensor continuously transmits data, as long as the reading unit is coupled to the sensor.

The components of the transponder can be formed from separate integrated circuit devices, but are preferably formed on a single integrated circuit chip that integrates the functions of all the components in a very compact package.

In use, shunt 12 is implanted into a patient suffering from hydrocephalus. Excess fluid in the lateral ventricle 24 of the patient is allowed to escape to a diversion site 26 by passing through ventricular catheter 30, through lumen 42, interacting with pressure transducer 52 and flow-rate transducer 56, passing through valve 34 and finally reaching the diversion site by way of diversion catheter 36.

During the course of the treatment, it may be desirable to measure CSF fluid rate and ICP within the shunt implanted in the patient. To do this, a clinician or physician moves interrogation antenna 64 of reader 14 near the location of the implanted sensor pod 32 so that RF energy from RF emitter 100 transmitted by interrogation antenna 64 can be received by loop antenna 62. When this happens, RF/DC converter 84 and voltage regulation block 86 converts the RF energy to a power supply that powers all the electrical components of the processing circuit 60 and transducers 52, 54, as necessary. Once powered up, the processing circuit 60 immediately reads outputted values of pressure from pressure transducer 52 and flow-rate values from flow-rate transducer 54. These values are each conditioned by signal conditioning blocks 70, 72, essentially digitizing the signals and then are sent to controller block (microprocessor) 76. Controller block 76 uses calibration information stored in memory 78 to adjust the values of the transducers outputs 52, 54 and further multiplexes the signals to prepare them for transmission. The controller 76 and absorption modulation block 82 work together to encrypt the multiplexed signals onto an RF carrier wave that is then transmitted, preferably as a single data message to and received by the adjacent interrogation antenna 64. This entire process occurs repeatedly many times a second and therefore can provide reader 14 with realtime data from transducers 52, and 54. Although it is desirable to transmit the data from the implanted sensor to the interrogation antenna 64 in a single data message, other transmission techniques may be employed to accomplish the same result. For example the data from each transducer, 52, 54 can be independently processed and transmitted separately within their own data message to a reader either simultaneously or sequentially. The reader could then collect the data streams as they are received from each transducer and use the information to calculate the desired flow rate, ICP, and brain compliance to be displayed.

Once the data is received by the interrogation antenna 64, the signal is decoupled and demultiplexed by decoupler block 104 and demulitplexer block 106 and sent to a data treatment block 108. As described above, data treatment block uses a barometric pressure input from barometer block 110 and some internal calculations to calculate real time CSF flow rate, ICP and brain compliance. These parameters are then sent to appropriate display circuitry (not shown) and finally displayed on display 112 for the clinician or physician to use immediately.

**Claims**

1. A shunt system (10) including a shunt assembly (12) and a reader assembly (14), said shunt assembly (12) for transferring fluids from a first location (24) within a patient to a second location and for monitoring said fluid transfer from a remote location, said shunt system (10) comprising:

   said shunt assembly (12) having:

   a conduit having a lumen (42) through which a fluid may flow, said conduit being implantable within said patient between said first (24) and second (26) locations;
   a pressure transducer (52) positioned on or within said conduit to measure a pressure of the fluid located within said lumen and generate a first signal in response to said measurement;
   a flow-rate transducer (54) positioned on or within said conduit to measure a rate of flow of the fluid flowing within said lumen (42) and generate a second signal in response to said measurement; and
   a telemetry circuit for transmitting said first and second signals;

   said reader assembly having:

   a remote reader (14) disposed outside of said patient for receiving said transmitted first and second signals; and a display (112) for visually conveying said first and second parameters based on said first and second signals; **characterised in that**
   said first and second signals are continuously transmitted when the telemetry circuit is inductively coupled to the remote reader (14).

2. The shunt assembly (12) of claim 1, wherein said lumen (42) further comprises a one way valve (34) so that fluid can only flow in one direction.

3. The shunt assembly (12) of any one of claims 1-2, wherein said first (52) and second electronic transducers (54) are (i) physically located adjacent to each other and/or (ii) provided on a common semiconductor substrate and/or (iii) located within a capsule (55) that is attached to said conduit adjacent to said lumen (42).

4. The shunt assembly (12) of any preceding claim, wherein said remote reader (14) further comprises a barometer that provides local barometric pressure measurements.

5. The shunt assembly (12) of claim 4, wherein said remote reader (14) uses said first and second signals and said barometric pressure measurements to calculate real-time values of ICP, CSF fluid flow, and brain compliance.

6. The shunt assembly (12) of any preceding claim, wherein said first (52) and second transducers (54) are electrically connected to said telemetry circuitry.

7. The shunt system (10) of claim 1, wherein said telemetry circuit is adapted to transmit said first and second signals in response to receiving an interrogation signal.

8. The shunt assembly (12) of claim 7, wherein said interrogation signal is a radio frequency signal and is used to inductively power said telemetry circuitry.

9. A method of monitoring the flow rate of CSF and the intra-cranial pressure within a shunt (22), said shunt having a conduit having a lumen (42) through which CSF may flow, an electronic pressure transducer (52) positioned on or within said conduit, an electronic flow rate transducer (54) positioned on or within said conduit, a telemetry circuit, said shunt having been implanted within a patient between one of the ventricles of the patient (24) and a diversion site (26);
measuring the pressure of the CSF located within said lumen (42) with said electronic pressure transducer (52); generating a first signal in response to said pressure measurement; measuring the flow rate of the CSF moving through said lumen (42) with said electronic flow rate transducer (54); and generating a second signal in response to said flow rate measurement; **characterised by** the telemetry circuit continuously transmitting said first and second signals when said telemetry circuit is inductively coupled with a remote reader (14) disposed outside the patient.

10. The method of claim 9, further comprising the step of: receiving an interrogation signal; and transmitting by telemetry said first and second signals in response to receiving an interrogation signal.

**Patentansprüche**

1. Shunt-System (10) mit einer Shunt-Anordnung (12) und einer Lesegerätanordnung (14), wobei die Shunt-Anordnung (12) zur Überführung von Flüssigkeiten von einem ersten Ort (24) innerhalb eines Patienten an einen zweiten Ort und zur Überwachung des Flüssigkeitstransfers von einem entfernten Ort dient, wobei das Shunt-System (10) das Folgende umfasst:
wobei die Shunt-Anordnung (12) das Folgende aufweist:

einen Kanal mit einem Lumen (42), durch das eine Flüssigkeit fließen kann, wobei der Kanal in dem Patienten zwischen den ersten (24) und zweiten (26) Orten implantierbar ist;
einen Druckwandler (52), der zum Messen eines Drucks der im Lumen befindlichen Flüssigkeit und zum Generieren eines ersten Signals als Reaktion auf die Messung auf oder in dem Kanal angeordnet ist;
einen Durchflusswandler (54), der zum Messen einer Durchflussrate der im Lumen (42) fließenden Flüssigkeit und zum Generieren eines zweiten Signals als Reaktion auf die Messung auf oder in dem Kanal angeordnet ist; und
eine Telemetrieschaltung zur Übertragung der ersten und zweiten Signale; wobei die Lesegerätanordnung das Folgende aufweist:

ein Remote-Lesegerät (14), das zum Empfangen der übertragenen ersten und zweiten Signals außerhalb des Patienten angeordnet ist; und eine Anzeige (112) zur visuellen Übermittlung der ersten und zweiten Parameter auf Basis der ersten und zweiten Signale; **dadurch gekennzeichnet, dass**
die ersten und zweiten Signale kontinuierlich übertragen werden, wenn die Telemetrieschaltung induktiv mit dem Remote-Lesegerät (14) gekoppelt ist.

**2.** Shunt-Anordnung (12) nach Anspruch 1, wobei das Lumen (42) ferner ein Einwege-Ventil (34) umfasst, so dass Flüssigkeit nur in einer Richtung fließen kann.

**3.** Shunt-Anordnung (12) nach einem der Ansprüche 1-2, wobei die ersten (52) und zweiten elektronischen Wandler (54) (i) physisch nebeneinander angeordnet sind und/oder (ii) auf einem gemeinsamen Halbleitersubstrat vorgesehen sind und/oder (iii) innerhalb einer Kapsel (55) angeordnet sind, die am Kanal neben dem Lumen (42) befestigt ist.

**4.** Shunt-Anordnung (12) nach einem der vorhergehenden Ansprüche, wobei das Remote-Lesegerät (14) ferner ein Barometer umfasst, das lokale barometrische Druckmessungen bereitstellt.

**5.** Shunt-Anordnung (12) nach Anspruch 4, wobei das Remote-Lesegerät (14) die ersten und zweiten Signale und die barometrischen Druckmessungen zur Berechnung von Echtzeitwerten von intrakraniellem Druck, Liquor-Flüssigkeitsströmung und Gehirn-Compliance verwendet.

**6.** Shunt-Anordnung (12) nach einem der vorhergehenden Ansprüche, wobei die ersten (52) und zweiten (54) Wandler elektrisch mit der Telemetrieschaltung verbunden sind.

**7.** Shunt-Anordnung (10) nach Anspruch 1, wobei die Telemetrieschaltung zur Übertragung der ersten und zweiten Signale als Reaktion auf den Empfang eines Abfragesignals angepasst ist.

**8.** Shunt-Anordnung (12) nach Anspruch 7, wobei das Abfragesignal ein Radiofrequenzsignal ist und zum induktiven Antreiben der Telemetrieschaltung verwendet wird.

**9.** Verfahren zur Überwachung der Liquorströmungsrate und des intrakraniellen Drucks in einen Shunt (22), wobei der Shunt einen Kanal mit einem Lumen (42), durch das Liquor fließen kann, einen elektronischen Druckwandler (52), der auf oder in dem Kanal angeordnet ist, einen elektronischen Durchflusswandler (54), der auf oder in dem Kanal angeordnet ist, und eine Telemetrieschaltung aufweist, wobei der Shunt in einem Patienten zwischen einem der Ventrikel des Patienten (24) und einer Umleitungsstelle (26) implantiert wurde;
Messung des Drucks des Liquors im Lumen (42) mit dem elektronischen Druckwandler (52);
Generierung eines ersten Signals als Reaktion auf die Druckmessung; Messung der Durchflussrate des durch das Lumen (42) fließenden Liquors mit dem elektronischen Durchflusswandler (54); und
Generierung eines zweiten Signals als Reaktion auf die Durchflussmessung; **dadurch gekennzeichnet, dass** die Telemetrieschaltung kontinuierlich die ersten und zweiten Signals überträgt, wenn die Telemetrieschaltung induktiv mit einem außerhalb des Patienten angeordneten Remote-Lesegerät (14) gekoppelt ist.

**10.** Verfahren nach Anspruch 9, ferner umfassend den Schritt des:

Empfangens eines Abfragesignals; und
Übertragens, mittels Telemetrie, der ersten und zweiten Signale als Reaktion auf den Empfang eines Abfragesignals.

**Revendications**

**1.** Système de dérivation (10) comprenant un ensemble de dérivation (12) et un ensemble de lecture (14), ledit ensemble de dérivation (12) étant destiné à transférer des fluides d'un premier emplacement (24) à l'intérieur d'un patient vers un second emplacement et à surveiller ledit transfert de fluide depuis un emplacement distant, ledit système de dérivation (10) comprenant :
ledit ensemble de dérivation (12) ayant :

un conduit ayant une lumière (42) à travers laquelle un fluide peut s'écouler, ledit conduit pouvant être implanté à l'intérieur dudit patient entre lesdits premier (24) et second (26) emplacements ;
un transducteur de pression (52) positionné sur ou à l'intérieur dudit conduit pour mesurer une pression du fluide situé à l'intérieur de ladite lumière et générer un premier signal en réponse à ladite mesure ;
un transducteur de débit (54) positionné sur ou à l'intérieur dudit conduit pour mesurer un débit du fluide s'écoulant dans ladite lumière (42) et générer un second signal en réponse à ladite mesure ; et
un circuit de télémétrie pour transmettre lesdits premier et second signaux ; ledit ensemble lecteur ayant :

un lecteur distant (14) disposé à l'extérieur dudit patient pour recevoir lesdits premier et second signaux transmis ; et un dispositif d'affichage (112) pour transmettre visuellement lesdits premier et second paramètres sur la base desdits premier et second signaux ; **caractérisé en ce que** lesdits premier et second signaux sont transmis en continu lorsque le circuit de télémétrie est couplé par induction au lecteur distant (14).

2. Ensemble de dérivation (12) selon la revendication 1, ladite lumière (42) comprenant en outre une soupape unidirectionnelle (34) de sorte que le fluide ne peut s'écouler que dans une seule direction.

3. Ensemble de dérivation (12) selon l'une quelconque des revendications 1-2, lesdits premier (52) et second transducteurs électroniques (54) étant (i) physiquement situés adjacents l'un à l'autre et/ou (ii) disposés sur un substrat semi-conducteur commun et/ou (iii) situés à l'intérieur d'une capsule (55) qui est fixée audit conduit adjacent à ladite lumière (42).

4. Ensemble de dérivation (12) selon n'importe quelle revendication précédente, ledit lecteur distant (14) comprenant en outre un baromètre qui fournit des mesures locales de pression barométrique.

5. Ensemble de dérivation (12) selon la revendication 4, ledit lecteur distant (14) utilisant lesdits premier et second signaux et lesdites mesures de pression barométrique pour calculer des valeurs en temps réel d'ICP, de débit de fluide CSF et de conformité cérébrale.

6. Ensemble de dérivation (12) selon n'importe quelle revendication précédente, lesdits premier (52) et second transducteurs (54) étant connectés électriquement auxdits circuits de télémétrie.

7. Système de dérivation (10) selon la revendication 1, ledit circuit de télémétrie étant adapté pour transmettre lesdits premier et second signaux en réponse à la réception d'un signal d'interrogation.

8. Ensemble de dérivation (12) selon la revendication 7, ledit signal d'interrogation étant un signal radiofréquence et étant utilisé pour alimenter par induction lesdits circuits de télémétrie.

9. Procédé de surveillance du débit de CSF et de la pression intracrânienne à l'intérieur d'une dérivation (22), ladite dérivation ayant un conduit ayant une lumière (42) à travers laquelle le fluide CSF peut circuler, un transducteur électronique de pression (52) placé sur ou dans ledit conduit, un transducteur électronique de débit (54) placé sur ou dans ledit conduit, un circuit télémétrique, ladite dérivation étant implantée à l'intérieur d'un patient entre un des ventricules du patient (24) et un site de diversion (26) ;
mesurant la pression du CSF situé à l'intérieur de ladite lumière (42) avec ledit transducteur de pression électronique (52) ;
générant un premier signal en réponse à ladite mesure de pression ; mesurant le débit du fluide CSF se déplaçant dans ladite lumière (42) avec ledit transducteur électronique de débit (54) ; et
générant un second signal en réponse à ladite mesure de débit ;
**caractérisé en ce que** le circuit de télémétrie transmet en continu lesdits premier et second signaux lorsque ledit circuit de télémétrie est couplé par induction à un lecteur distant (14) disposé à l'extérieur du patient.

10. Procédé selon la revendication 9, comprenant en outre l'étape de :

réception d'un signal d'interrogation ; et
transmission par télémétrie desdits premier et second signaux en réponse à la réception d'un signal d'interrogation.

**Figure 1**

**Figure 2**

**Figure 3**

Figure 4

**Figure 5**

DISPLAY $\sim$112

Barometer $\sim$110

De-MUX $\sim$106

Pressure Data

Flow Data

Data Treatment $\sim$108

Sensor Pressure

Compliance

Flow Rate

14

Decoupler $\sim$104

RF emitter $\sim$100

64

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4676255 A
- US 4660568 A
- US 4653508 A
- US 4593703 A
- US 4378809 A
- US 4281667 A
- US 4206761 A
- US 4206762 A
- WO 0121066 A
- EP 1050264 A
- EP 1491137 A
- US 7069779 B
- US 7181963 B
- US 7036369 B

**Non-patent literature cited in the description**

- **ZHENGNIAN TANG ; BRIAN SMITH ; JOHN H. SCHILD ; P. HUNTER PECKHAM.** Data Transmission from An Implantable Biotelemeter by Load-Shift Keying Using Circuit Configuration Modulator. *IEEE Transaction on Biomedical Engineering,* May 1995, vol. 42 (5